# EUROPEAN PATENT APPLICATION

(11) **EP 4 534 093 A1**
(43) Date of publication of application: **09.04.2025**
(21) Application number: 23816400.8
(22) Date of filing: 02.06.2023
(51) Int. Cl.: A61K 35/15, A61K 45/06, A61P 35/00, G01N 33/50

(54) **PHARMACEUTICAL COMPOSITION FOR PREVENTING OR TREATING CANCER, CONTAINING, AS ACTIVE INGREDIENT, MONOCYTES OR MACROPHAGES IN WHICH EXPRESSION OR ACTIVITY OF CD244 IS INHIBITED**

(30) Priority: 02.06.2022 KR 20220067313; 01.06.2023 KR 20230070870
(71) Applicant: Korea University Research and Business Foundation, Seoul 02841 (KR)
(72) Inventor: LEE, Kyung-Mi, Seoul 06001 (KR); KIM, Jeong Soo, Seoul 02829 (KR)
(74) Representative: Mewburn Ellis LLP
(86) International application number: PCT/KR2023/007574
(87) International publication number: WO 2023/234740

(57) **Abstract**

The present invention relates to a pharmaceutical composition for preventing or treating cancer, containing, as an active ingredient, monocytes or macrophages in which the expression or activity of CD244 is inhibited. It has been identified that CD244 inhibits differentiation from monocytes to macrophages, and monocytes or macrophages in which the expression or activity of CD244 is inhibited increase phagocytosis and antigen presentation such that tumor growth is inhibited, and induce antigen-specific activation of T cells, and thus the present invention can be effectively used as a composition for preventing or treating cancer.

## Description

### Technical Field

The present disclosure relates to a pharmaceutical composition for preventing or treating a cancer, including, as an active ingredient, monocytes or macrophages with suppressed CD244 expression or activity.

### Background Art

Immune exhaustion is caused by an excessive immune response or immunosuppressive surroundings, and it is known that anti-tumor immune responses and reduced expression of inflammatory cytokines lead to formation of an immunosuppressive tumor microenvironment. Immune exhaustion takes place mainly through molecular mechanisms by immune checkpoint receptors PD-1, CTLA-4, LAG-3, and TIM-3 that are expressed by T cells. Recently, antibody therapeutics that inhibit signaling of PD-1, PD-L1, and CTLA-4 have been developed as immune checkpoint inhibitors (ICIs) to treat multiple cancers that are refractory to existing standard chemotherapy. However, these therapeutics are effective only in a small number of patients who meet the limited genotypes and tumor microenvironment due to various factors such as the immunosuppressive tumor microenvironment of solid tumors, tumor cell polymorphism, and a lack of tumor-infiltrating immune cells.

CD244 (SLAMF4, 2B4) is an immune cell receptor belonging to the signaling lymphocyte activation molecule family (SLAMF). CD244 is a unique receptor capable of performing two functions, such as activation and inhibition of immune responses, depending on which signaling molecule it binds on NK cells and CD8 T cells. Studies that have been conducted recently have revealed that, in the tumor microenvironment, CD244 inhibits NK cells and CD8 T cells to maintain their exhausted state. The intracellular domain of CD244 consists of four immunoreceptor tyrosine-based switch motifs (ITSMs), with a capability of binding to signaling molecules such as SAP, EAT-2, SHP1, SHP2, and SHIP-1. CD244 is known to transmit immunoactive signals when combined with SAP and immunosuppressive signals when combined with SH2 phosphatase. The signal that CD244 transmits depends on the relative expression level of signaling molecules and their competitive binding.

Monocytes and macrophages are important cells that bring innate and adaptive immune responses together, sense the surroundings, and determine innate and adaptive immune responses. Many recent studies have focused on monocytes and macrophages as important cells that form "cold tumors", which refer to tumors that barely respond to immunotherapy. In a study of immune cells in patients with advanced liver cancer, it was discovered that a large number of monocytes and macrophages differentiated from monocytes infiltrated tissues around the tumor, which is associated with a decrease in NK cell counts in the tumor and immunoactive cytokines such as TNF-a and IFN-g. In addition, the same study showed that when CD48, which is highly expressed in tumor-infiltrating monocytes, binds to CD244 in NK cells, NK cells become severely exhausted and unable to function properly. Though CD244 is expressed not only in CD8 T cells and NK cells, but also in various myeloid immune cells such as monocytes and macrophages, its role in these cells is unclear.

Therefore, contrary to previous studies, the present inventor has attempted to find out what function CD244 expressed in monocyte-macrophages performs through interactions with CD48 that is expressed by surrounding immune cells.

### Disclosure of the Invention

### Technical Goals

An object of the present disclosure is to provide a pharmaceutical composition for preventing or treating a cancer, including, as an active ingredient, monocytes or macrophages with suppressed CD244 expression or activity.

### Technical Solutions

To achieve the above object, the present disclosure provides a pharmaceutical composition for preventing or treating a cancer, including, as an active ingredient, monocytes or macrophages with suppressed CD244 expression or activity.

In addition, the present disclosure provides a pharmaceutical composition for co-administration for preventing or treating a cancer, including monocytes or macrophages with suppressed CD244 expression or activity; and an anticancer drug as active ingredients.

In addition, the present disclosure provides a composition for enhancing efficacy of an anticancer drug, including, as an active ingredient, monocytes or macrophages with suppressed CD244 expression or activity.

In addition, the present disclosure provides a method of preventing or treating a cancer, including administrating monocytes or macrophages with suppressed CD244 expression or activity to a subject, including a human, alone or in combination with an anticancer drug.

In addition, the present disclosure provides a composition for inducing differentiation from monocytes to macrophages, including a CD244 inhibitor as an active ingredient.

In addition, the present disclosure provides a method of screening an inducer for differentiation from monocytes to macrophages, including treating cells with candidate substances to analyze suppression of CD244 expression or activity.

### Advantageous Effects

According to the present disclosure, it was found that CD244 suppresses differentiation from monocytes to macrophages, and monocytes and macrophages with suppressed CD244 expression or activity inhibit tumor growth by increasing phagocytosis and antigen presentation to induce antigen-specific activation of T cells, such that the present disclosure may be useful as a composition for preventing or treating a cancer.

### Brief Description of Drawings

FIG. 1 shows results of analyzing changes in CD244 expression in monocytes upon tumor induction and an anticancer effect using a monocyte-specific CD244-deficient mouse model.
FIG. 2 shows results of analyzing intratumor T cell activity and tumor antigen specificity between Flox and cKO mice.
FIG. 3 shows results of analyzing an intratumor monocytes-macrophage proportion between Flox and cKO mice and whether there is a change in monocyte-macrophage differentiation under CD244 deficiency.
FIG. 4 shows results of analyzing whether CD244 affects functions such as M1 macrophage polarization, antigen presentation, and phagocytosis using a CD244-deficient mouse model.
FIG. 5 shows results of analyzing whether expression of CD244 is induced by ER stress in monocytes through single-cell transcriptome analysis data and cell experiments.
FIG. 6 shows results of analyzing whether CD244 regulates monocyte-macrophage differentiation through autophagy.
FIG. 7 shows results of analyzing whether the efficacy of immune checkpoint inhibitors increases when CD244 is removed in a monocyte-macrophage-specific manner or CD244-deficient macrophages are administered.
FIG. 8 shows results of analyzing whether there is a difference in functions and survival rate of monocyte-macrophage and T cells between patients with high and low CD244 expression levels in melanoma patients through single-cell transcriptome analysis data.
FIG. 9 shows results of analyzing whether CD244 regulates monocyte-macrophages as in melanoma in patients with colorectal cancer, non-small cell lung cancer, and glioblastoma through single-cell transcriptome analysis data.

### Best Mode for Carrying Out the Invention

Hereinafter, the present disclosure will be described in more detail.

The present disclosure provides a pharmaceutical composition for preventing or treating a cancer, including, as an active ingredient, monocytes or macrophages with suppressed CD244 expression or activity.

The pharmaceutical composition may suppress cancer growth by increasing phagocytosis or antigen presentation.

In addition, the pharmaceutical composition may induce antigen-specific activation of T cells, thereby increasing the expression of IFN-g.

The monocyte may include, but is not limited to, one or more markers selected from the group consisting of CD11b+, CD14+, CD16-, Ly6G-, and Ly6Chi.

The macrophage may include, but is not limited to, one or more markers selected from the group consisting of CD11b+, CD14+, CD16+, CD68+, Ly6G-, Ly6Clo, and F4/80+.

The cancer may be one or more selected from the group consisting of, but is not limited to, stomach cancer, colon cancer, breast cancer, lung cancer, non-small cell lung cancer, bone cancer, pancreatic cancer, skin cancer, head cancer, head and neck cancer, melanoma, uterine cancer, ovarian cancer, colon cancer, small intestine cancer, rectal cancer, anal cancer, fallopian tube cancer, endometrial cancer, cervical cancer, vaginal cancer, vulvar cancer, Hodgkin's disease, esophageal cancer, lymphoma, bladder cancer, gallbladder cancer, endocrine cancer, prostate cancer, adrenal cancer, soft tissue sarcoma, urethral cancer, penile cancer, chronic or acute leukemia, lymphocytic lymphoma, renal cancer, ureteral cancer, renal pelvic cancer, blood cancer, brain cancer, central nervous system (CNS) tumors, spinal cord tumors, brainstem gliomas, and pituitary adenomas.

The pharmaceutical composition of the present disclosure may be prepared in the form of a unit dose by formulation using a pharmaceutically acceptable carrier or prepared by encapsulating into a multi-capacity container, in accordance with a method that may be easily carried out by a person skilled in the art to which the disclosure pertains.

The pharmaceutically acceptable carrier is commonly used in preparation, including, but is not limited to, lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil. The pharmaceutical composition of the present disclosure may further include lubricants, humectants, sweeteners, flavoring agents, emulsifiers, suspensions, and preservatives, in addition to the above ingredients.

In the present disclosure, the content of the additives included in the pharmaceutical composition is not particularly limited and may be appropriately adjusted within a content range used in the conventional preparation.

The pharmaceutical composition may be formulated in the form of one or more external skin agents selected from the group consisting of, but are not limited to, injectable formulations such as aqueous solutions, suspensions, and emulsions, pills, capsules, granules, tablets, creams, gels, patches, sprays, ointments, plasters, lotions, liniments, pastas, and cataplasmas.

The pharmaceutical composition of the present disclosure may include a pharmaceutically acceptable carrier and diluent that are additionally present for formulation. The pharmaceutically acceptable carrier and diluents include, but are not limited to, excipients such as starch, sugars, and mannitol, fillers and extenders such as calcium phosphate, cellulose derivatives such as carboxymethylcellulose and hydroxypropylcellulose, binders such as gelatin, alginate, and polyvinyl pyrrolidone, lubricants such as talc, calcium stearate, hydrogenated castor oil, and polyethylene glycol, disintegrating agents such as povidone and crospovidone, and surfactants such as polysorbate, cetyl alcohol, and glycerol. The pharmaceutically acceptable carrier and diluent may be biologically and physiologically friendly to a subject. Examples of diluents may include, but are not limited to, brine, water-soluble buffers, solvents, and/or dispersion media.

The pharmaceutical composition of the present disclosure may be administered orally or parenterally (e.g., applied intravenously, subcutaneously, intraperitoneally, or topically) depending on the desired method. When administered orally, it may be formulated as tablets, troches, lozenges, water-soluble suspensions, oil-based suspensions, powder preparation, granules, emulsions, hard capsules, soft capsules, syrups, and elixirs. When administered parenterally, it may be formulated as injection solutions, suppositories, powders for respiratory inhalation, aerosols for sprays, ointments, powders for application, oils, and creams.

The dosage of the pharmaceutical composition of the present disclosure may vary depending on the patient's condition, weight, age, sex, health status, dietary constitution specificity, nature of the preparation, severity of disease, administration time for composition, mode of administration, duration or interval of administration, excretion rate, and drug form, and may be appropriately selected by a person skilled in the art. For example, the dosage may be in the range of about 0.1 to 10,000 mg/kg but is not limited thereto, while it may be administrated in divided doses from one to several times a day.

The pharmaceutical composition may be administered orally or parenterally (e.g., intravenously, subcutaneously, intraperitoneally, or topically) depending on a desired method. A pharmaceutically effective amount and effective dosage of the pharmaceutical composition of the present disclosure may vary depending on preparation methods, administration modes, administration duration, and administration routes of the pharmaceutical composition, and those skilled in the art may easily determine and prescribe the dosage that is effective for desired treatment. Administration of the pharmaceutical composition of the present disclosure may be conducted once a day or several times in divided doses.

In addition, the present disclosure provides a pharmaceutical composition for co-administration for preventing or treating a cancer, including monocytes or macrophages with suppressed CD244 expression or activity; and an anticancer drug as active ingredients.

The anticancer drug may be, but is not limited to, one or more anticancer drugs selected from the group consisting of a cytotoxic anticancer drug, a targeted anticancer drug, and an immunotherapeutic drug.

The cytotoxic anticancer drug may be, but is not limited to, one or more selected from the group consisting of doxorubicin, cyclophosphamide, temozolomide, irinotecan, cisplatin, vinblastine, vincristine, actinomycin-D, 5-fluorouracil, docetaxel, cabazitaxel, paclitaxel, and pembrolizumab.

The targeted anticancer drug may be, but is not limited to, one or more selected from the group consisting of trametinib, vemurafenib, alpelisib, dactolisib, Gefitinib, Erlotinib, Lapatinib, Sunitinib, Sorafenib, Crizotinib, Dabrafenib, Trastuzumab, Cetuximab, Bevacizumab, Panitumumab, Ipilimumab, Pertuzumab, Tofacitinib, Imatinib, Bortezomib, Ofatumumab, and Alemtuzumab.

The immunotherapeutic drug may be, but is not limited to, one or more selected from the group consisting of anti-PD-1, anti-PD-L1, anti-CTLA-4, anti-LAG-3, anti-TIGIT, anti-TIM-3, anti-GITR, CAR-T, and NK cells.

In addition, the present disclosure provides a composition for enhancing efficacy of an anticancer drug, including, as an active ingredient, monocytes or macrophages with suppressed CD244 expression or activity.

In addition, the present disclosure provides a method of preventing or treating a cancer, including administrating monocytes or macrophages with suppressed CD244 expression or activity to a subject, including a human, alone or in combination with an anticancer drug.

In addition, the present disclosure provides a composition for inducing differentiation from monocytes to macrophages, including a CD244 inhibitor or an inhibitor of CD48 which is a ligand of CD244 as an active ingredient.

The CD244 inhibitor or the CD48 inhibitor may be selected from the group consisting of compounds, peptides, peptide mimetics, aptamers, antibodies, and natural substances that specifically bind to a CD244 or CD48 protein, or selected from the group consisting of small interfering RNAs (siRNAs), antisense nucleotides, and short hairpin RNAs (shRNAs) that complementarily bind to an mRNA of a CD244 or CD48 gene.

The CD244 inhibitor may be, but is not limited to, human anti-CD244 monoclonal antibodies [ThermoFisher Scientific: #16-2449-81, BioLegend: #329502, R&D SYSTEMS: MAB10393-SP, BioVision: A1073-100, BD Biosciences: #550814] or mouse anti-CD244 monoclonal antibodies [ThermoFisher Scientific: #14-2441-82, BioLegend: #133501, BD Biosciences: #557451].

The CD48 inhibitor may be, but is not limited to, human anti-CD48 monoclonal antibodies [ThermoFisher Scientific: #16-0489-81, BioLegend: #336702, R&D SYSTEMS: MAB36441-SP, BD Biosciences: #555758] or mouse anti-CD48 monoclonal antibodies [BioXcell: #BE0147, BioLegend : #103453, BD Biosciences: #553682].

In addition, the present disclosure provides a method of screening an inducer for differentiation from monocytes to macrophages, including treating cells with candidate substances to analyze suppression of expression or activity of CD244 or CD48 which is a ligand thereof.

### Modes for Carrying Out the Invention

Hereinafter, the present disclosure will be described in more detail through examples to help understanding of the present disclosure. However, examples below are merely intended to illustrate the present invention, and the scope of the present disclosure is not limited to the following examples. Examples of the present disclosure are provided to more completely explain the present disclosure to those with ordinary skill in the art.

### [Experimental Example 1] Preparation of an animal model

All experiments using animals were approved by the Institutional Animal Care and Use Committee at Korea University (Approval Number: KUIACUC-2019-0101) and followed the guidelines and regulations of the Institutional Animal Care and Use Committee at Korea University.

Wild-type (WT) C57BL/6 mice were used as a control group for CD244-deficient mice and were purchased from Orient Bio (Orient Bio. Inc., Seongnam, South Korea). KO is CD244^{-/-} with a C57BL/6 background, and the mice are C57BL/6 CD244^{-/-} with CD244 deficiency in all cells. Flox, a C57BL/6 LysM^{+/+}-CD244^{fl/fl} mouse, was used as the control group with the same CD244 expression as the WT mouse and was custom-made by Cyagen Biosciences. cKO is LysM^{cre/+}-CD244^{fl/fl} and a monocyte-macrophage-specific CD244-deficient mouse, CD244^{fl/fl} mice were crossed with Lyz2^{cre+} (B6.129P2-Lyz2tm1(cre)Ifo/J) mice purchased from Jackson Laboratory to produce CD244^{fl/fl} and CD244^{fl/fl}-Lyz2^{cre+/-} mice, and littermate control mice were used in the CD244 conditional KO mouse experiment. Female mice aged 5~10 weeks were used in the experiment, and all mice were bred and maintained in Specific Pathogen Free Facility in Korea University.

The B16F10 melanoma cell line was purchased from ATCC. For subcutaneous injection, 1 × 10⁶ B16F10 cells were injected into the lateral abdomen of mice, and the formation of tumors with a diameter of 1cm was observed within 1~3 weeks of injection. For a co-administration experiment of bone marrow-derived macrophages (BMDM) and anti-PD-L1 antibody, bone marrow cells of WT and CD244 KO mice were cultured in RPMI medium containing 50 ng/ml recombinant M-CSF (Peprotech) for 6 days. WT C57BL/6 mice were intraperitoneally injected with 1 × 10⁶ WT and CD244 KO BMDM and 200 µg of anti-PD-L1 (BioXcell: #BE0101) and isotype Rat IgG2ak antibody (BioXcell) after 7 and 10 days of B16F10 tumor induction, respectively.

### [Experimental Example 2] Preparation of cells

After obtaining single cell suspension from the spleen and bone marrow, red blood cells were removed using ammonium chloride lysis buffer. In the single cell suspension of tumor tissues, immune cells were isolated and used in the experiment by mouse tumor dissociation kit and percoll density gradient separation according to the manufacturer's recommendations (Miltenyi Biotec and GE Healthcare).

### [Experimental Example 3] Isolation of CD11b+ cells and monocytes

For CD11b+ cell isolation, tumor single cell suspension was labeled with biotin-anti-CD11b antibody (Biolegend) and streptavidin-magnetic beads (Miltenyi biotec) and isolated using MACS LS column (Miltenyi Biotec). Similarly, to isolate monocytes, a monocyte isolation kit (Miltenyi Biotec) was used in bone marrow cells.

### [Experimental Example 4] Differentiation of BMDM and tumor invasive monocytes

CD11b+ cells isolated from isolated monocytes, total bone marrow cells, or tumor lysate were cultured by treating 50 ng/ml recombinant M-CSF (Peprotech) in RPMI (Wellgene) supplemented with 10% FBS, 1% penicillin/streptomycin, 1% non-essential amino acids (NEAA), 10 mM HEPES, and 20 µM 2-mercapoethantol. In some experiments, B16F10 tumor explant supernatant was used at 20% v/v.

### [Experimental Example 5] Flow cytometry

In general, up to 1 × 10⁶ cells were cultured with Fc-blocking antibody (anti-CD16/CD32 antibody, BioXcell: #BE0307) for 5 minutes at room temperature, surface protein staining was performed in a 4°C dark room for 30 minutes, and every centrifugation for washing was performed at 1700 rpm for 5 minutes. For intracellular IFN-gamma staining, isolated CD45+ or CD8+ cells of tumor or TDLN were reactivated by co-culture with B16F10 melanoma cells that are gamma-irradiated at 10gy along with brefeldin-A for 16 hours. The cells were then subjected to surface staining, punctured using a BD fixation/permeabilization kit as recommended by the manufacturer, and then stained intracellularly with PE-anti-IFN-g antibody (BD Biosciences). Data collected using FACS Canto II Flow Cytometry (BD Biosciences) was analyzed by FlowJo software (Tristar).

### [Experimental Example 6] Quantitative PCR

Total RNA from tissues and cells was extracted with TRIzol reagent (Invitrogen). cDNA was synthesized using a TOPscript^{™} cDNA synthesis kit (Enzynomics). Real-time PCR was performed using SYBR green (Bio-rad) on a StepOne Plus TM (ApplidBiosystems) instrument. Gene expression was normalized based on the expression level of GAPDH mRNA, and relative expression levels were calculated according to the ΔΔCT method.

### [Experimental Example 7] ELISPOT

5 × 10⁴ lymph node cells obtained from OT-1 transgenic mice were co-cultured with BMDM and OVA peptides (SIINFEKL; 1µg/ml) on the third day of differentiation in various ratios. Cells were plated in mouse IFN-g ELISPOT PLUS kit (ALP) (MABTECH), followed by detection of IFN-g spot via ELISPOT reader system (Autoimmun Diagnostika GmbH) after 48 hours.

### [Experimental Example 8] Statistical analyses

All data were presented as mean ± standard error (S.E.M). Statistical significance was determined by unpaired two-tailed t-test and two-way ANOVA. Significance was defined as *p<0.05, **p<0.01, and ***p<0.001. All analyses were performed using Prism 7.0 software (GraphPadSoftware, Inc).

### [Experimental Example 9] Antigen presentation assay

Two days after BMDM differentiation, 1 mg/ml of whole-OVA protein was added. MHCI complexes bound with OVA peptides and MHCII were labeled with anti-H-2kb-SIINFEKL antibody (Biolegend: #141604) and anti-MHCII antibody (Biolegend: #107622), followed by measurement of expression levels by flow cytometry.

### [Experimental Example 10] Phagocytosis assay

Two days after BMDM differentiation, gamma-irradiated B16F10 cells stained with 5 µM CFSE were co-cultured for 24 hours. Using flow cytometry, CFSE fluorescence was detected via 488/519 channel in CD45+, CD11b+, Ly6Chi, and F4/80low monocytes and CD45+, CD11b+, Ly6Clow, and F4/80high macrophages.

### [Experimental Example 11] Fluorescence microscopy

BMDM cells cultured in a confocal dish were fixed, and the cell membrane was perforated with 4.2% paraformaldehyde solution and stained with anti-LC3B antibody (abcam : #ab51520) (1:200) and Hoechst 33342 (Invitrogen : #H3570) (1000:1). After 2 hours of incubation, cells were washed, stained with Alexa555-Rabbit IgG secondary antibody (Invitrogen: #A27039), and incubated for 1 hour at room temperature. All images were recorded with an LSM700 confocal laser scanning microscope (Carl Zeiss) equipped with a 63X oil-immersed lens.

### [Experimental Example 12] Analysis of single-cell RNA sequencing data

Analysis was conducted on data from scRNA seq studies on changes in responsiveness to immune checkpoint blockade treatment and immune cells. Clustering of 5928 cells was performed in the pre-administration cohort of the immune checkpoint blockade using the Seurat v4 R package. Afterwards, patients were classified into CD244 high and low expressors depending on the average expression level of CD244 in monocyte-macrophages of patients. Only genes with relatively high mean expression and high expression differences between cells were selected to be used for downstream clustering analysis. Principal component analysis (PCA) was used for dimensional reduction, and the number of main components was calculated using the built-in JackStraw function. T-distributed stochastic neighbor embedding (t-SNE) and uniform manifold approximation and projection (UMAP) were used for two-dimensional data visualization. The FindMarker function built in the Seurat package was used to identify differentially expressed genes. From the results of the Seurat package, genes with a P value of less than 0.05 were considered as genes that are differentially expressed. For differentially expressed genes and gene expression ranks of each population, analysis on the signaling pathway and gene signature was performed through IPA software (QIAGEN Inc.) and fgsea R package.

### [Example 1] Analysis of an anticancer effect through CD244 inhibition on a mouse melanoma model

To determine whether CD244 expressed in monocytes has an impact on tumor progression, identified was whether CD244 expression in monocytes in tumors was elevated. A tumor model was constructed by subcutaneously injecting 1 × 10⁶ B16F10 cells, mouse allogeneic melanoma cells, into the lateral abdomen of C57BL/6 mice, and after 14 days, the expression level of CD244 by the immune cells in the tumor was compared with the intradermal immune cells of normal C57BL/6 mice. As a result, natural killer cells (hereinafter, referred to NK cells) and dendritic cells (hereinafter, referred to as DCs) showed high CD244 expression regardless of the presence of tumor, while monocytes rarely showed CD244 expression in normal skin tissue, and CD244 expression was significantly increasedafter tumor induction (FIG. 1a-A).

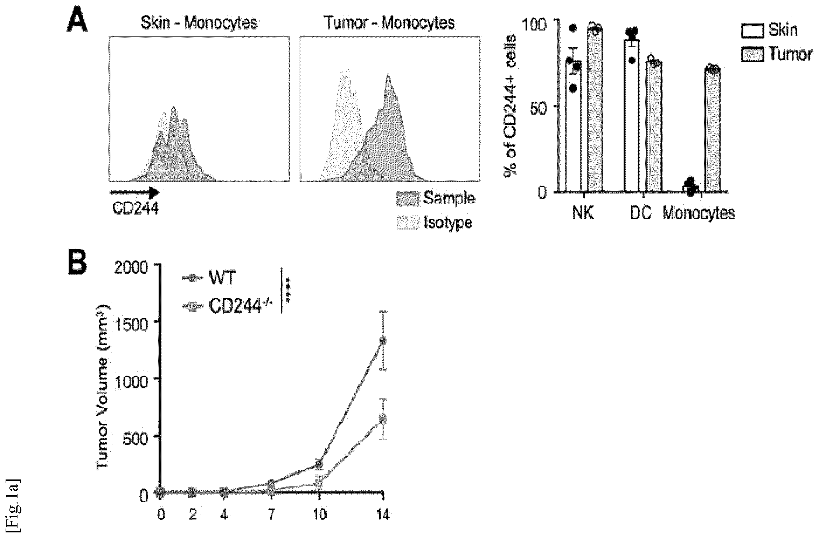

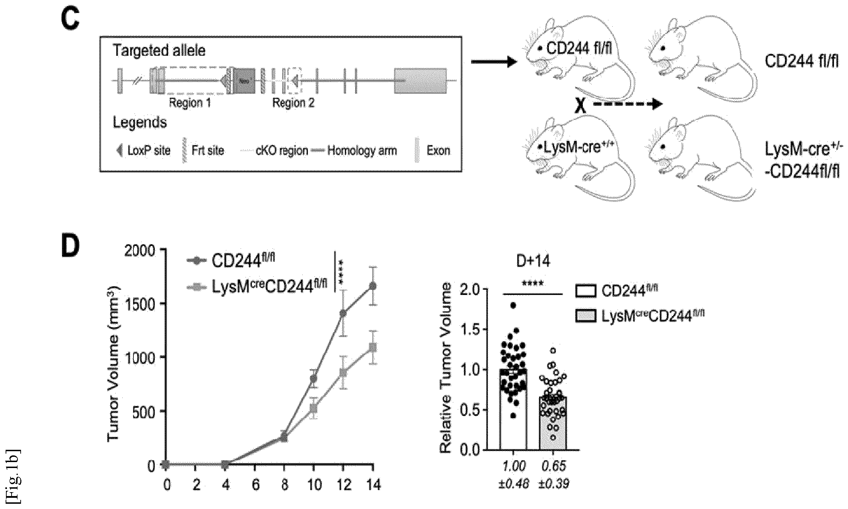

In addition, in order to determine whether the elevated expression of CD244 in monocytes after tumor induction affects tumor growth, a tumor model was constructed by subcutaneous injection of 1 × 10⁶ B16F10 into the lateral abdomen of C57BL/6 or C57BL/6 CD244^{-/-} mice, and the tumor growth was observed for 14 days. The tumor size was calculated, using vernier calipers, by width × length × height/2 of a tumor tissue. As a result, it was found that tumor growth was significantly inhibited in CD244^{-/-} mice (FIG. 1a-B).

In addition, since CD244 is expressed in a variety of immune cells other than monocytes, in order to determine more specific effects of CD244 expressed in monocytes, by crossing LysM-cre mice in which Cre recombinase is expressed along with monocyte-macrophage-specific LysM gene and CD244 flox mice attached with a flox sequence which is a gene sequence that can be cleaved by Cre recombinase before and after the CD244 gene, the monocyte-macrophage-specific CD244-deficient mice were constructed (FIG. 1b-C). Melanoma was induced in the control group CD244^{fl/fl} (hereinafter, referred to as Flox) and the experimental group LysM^{cre}-CD244^{fl/fl} (hereinafter, referred to as cKO) in the same way as FIG. 1a-B. As a result, it was found that tumor growth was significantly inhibited in the experimental group monocyte-macrophage-specific CD244-deficient mice (FIG. 1b-D).

### [Example 2] Analysis of changes in T cell activity in CD244-deficient monocytes/macrophages

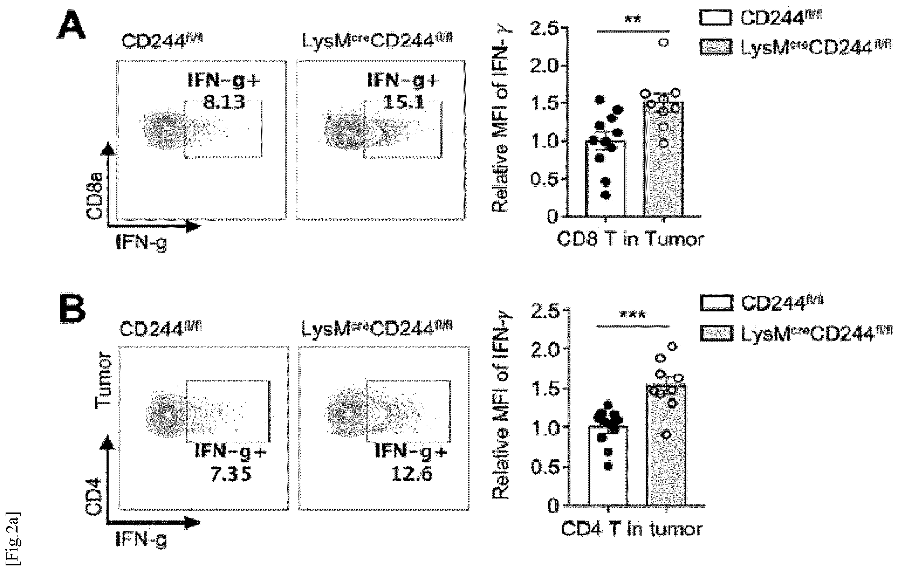

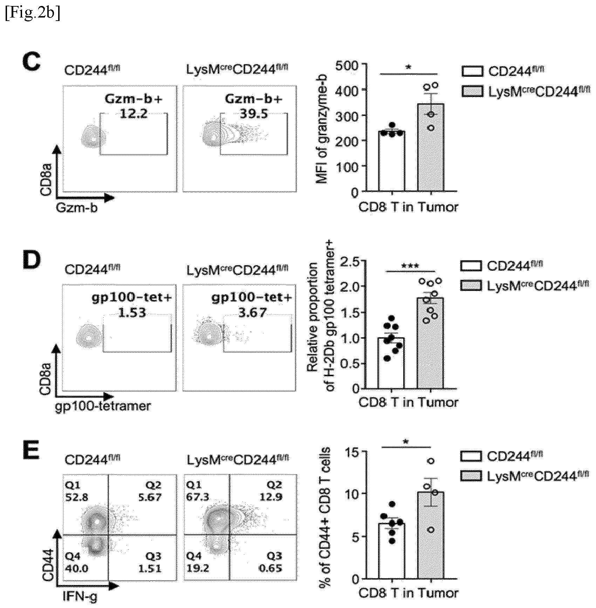

In order to determine how CD244 expressed in monocytes-macrophages suppresses the anti-tumor immune response, the activity of T cells, the most representative anti-tumor immune response, was identified. Melanoma was induced in Flox and cKO mice in the same way as FIG. 1b-D, and tumors were extracted after 14 days and analyzed by flow cytometry. As a result, there was no difference in the ratio of CD8 and CD4 T cells to NK cells, but it was found that CD8 and CD4 T cells expressed more IFN-g, which plays a pivotal role in the anti-tumor immune response in cKO mice, than the control group (Flox) (FIG. 2a). In addition, it was found that expression of granzyme-b, a protein that directly kills tumor cells, was significantly increased in CD8 T cells of cKO mice (FIG. 2b-C). It was determined, by staining with H-2Db gp100 tetramer, that this difference was due to an increase in antigen-specific CD8 cells expressing T cell receptors that are specific to melanoma antigen gp100 in cKO mice (FIG. 2b-D). In addition, the expression of CD44, a marker of memory T cells, was found to be higher in CD8 T cells of cKO mice (FIG. 2b-E).

### [Example 3] Analysis of changes in monocyte/macrophage distribution and differentiation in CD244-deficient mice

After extracting the tumors of the control group Flox mice and the experimental group cKO mice, the distribution of tumor-infiltrating immune cells was identified through flow cytometry. As a result, there was no difference in the proportion of CD11b+ cells which are total myeloid immune cells, neutrophils, and DC cells, but a significant decrease in monocytes and a significant increase in macrophages were observed in cKO mice (FIG. 3a-A).

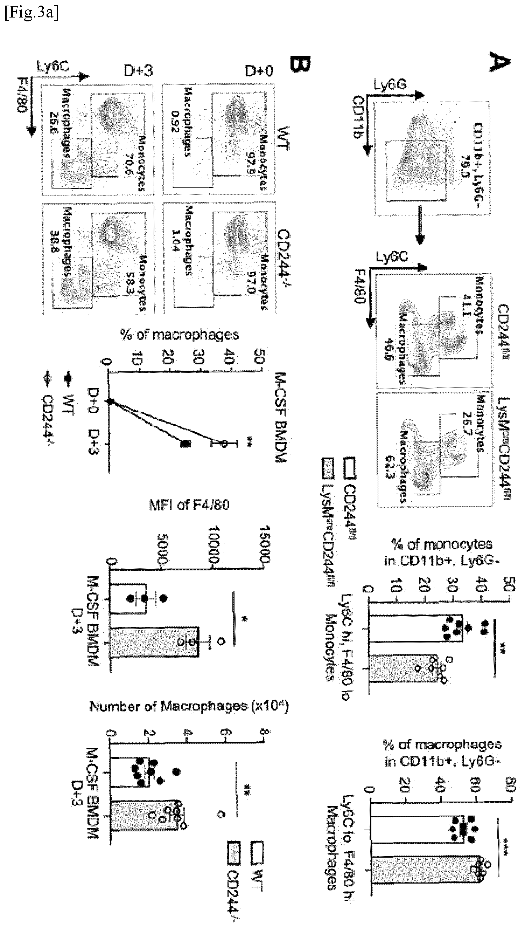

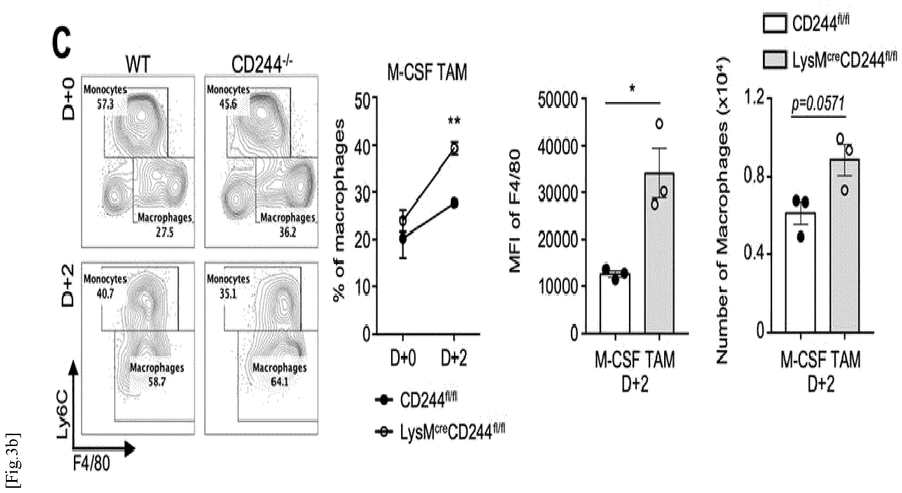

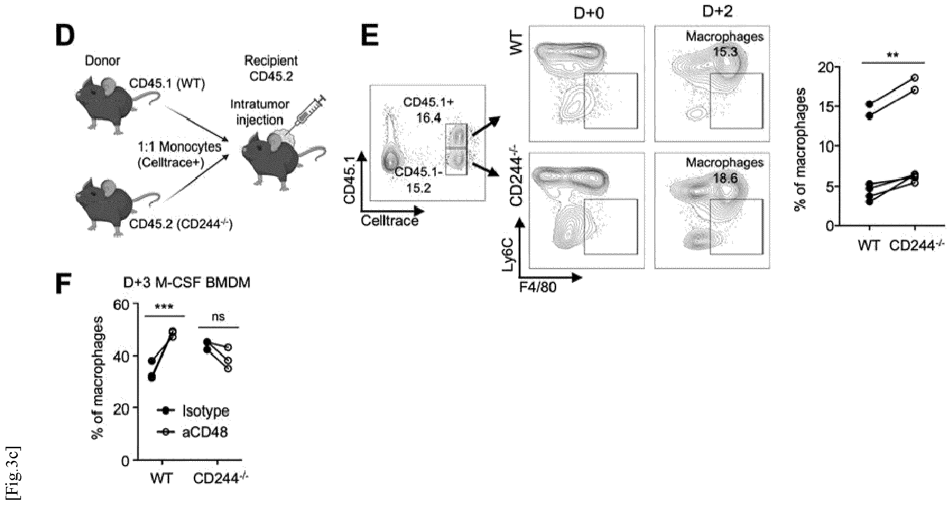

To determine whether CD244 regulates the process of differentiation of monocytes into macrophages, a BMDM experiment was performed, in which differentiation is induced by treating bone marrow cells of C57BL/6 or C57BL/6 CD244^{-/-} mice with M-CSF. After 3 days, as a result of measuring the proportion and number of macrophages, macrophages of CD244^{-/-} BMDM increased significantly compared to the control group (WT) (FIG. 3a-B). In addition, with a view that the physiological characteristics of monocytes in the bone marrow and monocytes in the tumor are significantly different, it was found that the proportion and number of macrophages also increased significantly when M-CSF was treated after the isolation of the monocytes in the tumor (FIG. 3b).

In addition, in order to directly find out whether CD244 regulates monocyte-macrophage differentiation even in the tumor microenvironment, monocytes were isolated from the bone marrow of C57BL/6 CD45.1 WT and C57BL/6 CD45.2 CD244^{-/-} mice, stained with cell trace far red, and mixed in a 1:1 ratio, followed by direct injection into the tumor of C57BL/6 CD45.2 mice (FIG. 3c-D). After 24 hours, as a result of checking CD45.1 WT and CD45.2 CD244^{-/-} cells stained with cell trace far red in the tumor, it was found that a significant amount of CD244^{-/-} monocytes were differentiated into macrophages (FIG. 3c-E).

To determine whether CD244 inhibits monocyte-macrophage differentiation through interaction with the ligand CD48 and sub-signaling, an anti-CD48 antibody (Biolegend: #103453) that blocks CD48, the ligand of CD244, was treated in the bone marrow-derived macrophage differentiation experiment. As a result, it was found that the number of macrophages did not change significantly in CD244^{-/-}, but increased remarkably in the WT mice (FIGS. 3c-F).

### [Example 4] Analysis of the mechanism of inhibiting adaptive immune responsiveness of CD244-deficient monocytes/macrophages

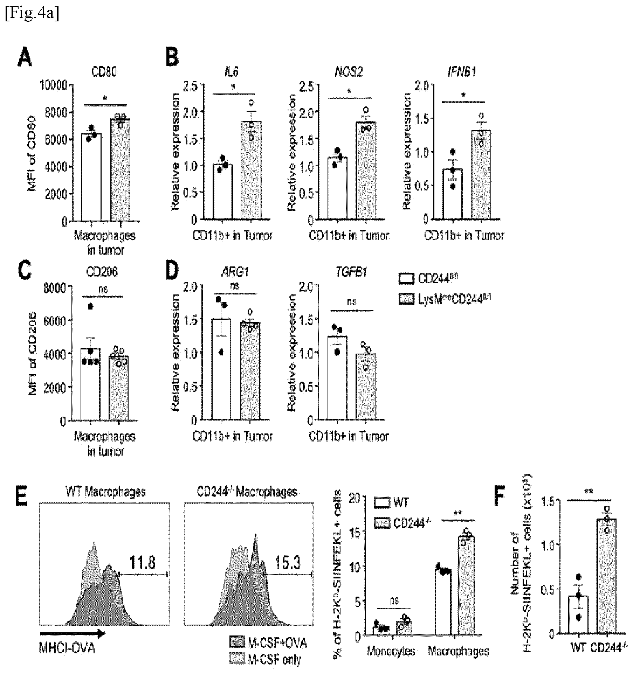

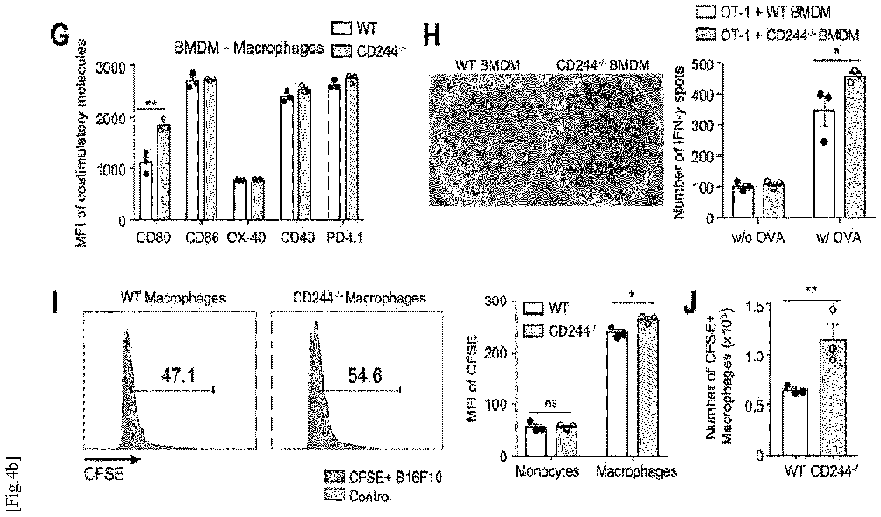

To determine how CD244 inhibits the adaptive immune responsiveness of monocytes and macrophages, tumors from control group Flox mice and experimental group cKO mice were extracted, and then markers of M1 and M2 macrophages were analyzed through flow cytometry and real-time PCR. As a result, the expression of M1 macrophage markers (CD80, IL-6, NOS2, and IFNB1) increased in the experimental group (cKO) compared to the control group (Flox) (FIG. 4a-A and FIG. 4a-B), while the M2 macrophage markers (CD206, ARG1, TGFB 1) showed no significant change in expression (FIG. 4a-C and FIG. 4a-D).

In addition, in order to determine whether antigen presentation, which is a key function of M1 macrophages, was regulated by CD244, the BMDM that was differentiated for 2 days was treated with an ovalbumin (hereinafter, referred to ova) protein and cultured for 24 hours to determine the degree of antigen presentation. As a result, the expression of the MHCI-ova complex was significantly increased in CD244-deficient macrophages (FIG. 4a-E), and the number of macrophages expressing the MHCI-ova complex was significantly increased in BMDM of CD244-deficient mice compared to the WT mice in combination with the increase in the number of macrophages in the CD244-deficient mice (FIG. 4a-F). In addition, when checking whether CD244 regulates the expression of co-stimulatory molecules essential for antigen presentation, it was identified that the expression of CD80 was high in CD244-deficient macrophages (FIG. 4b-G). In order to determine whether the increase in antigen-specific CD8 T cells in the tumor of cKO mice was due to inhibition of the antigen presentation of macrophages by CD244, OT-1 T cells that recognize ova was co-cultured with bone marrow-derived macrophages differentiated in vitro, and when ova peptides were added, it was found that T cell-specific IFN-g secretion increased when co-cultured with CD244-deficient macrophages (FIG. 4b-H).

Next, in order to determine the degree of occurrence of phagocytosis, which is an important mechanism in which macrophages are involved in antitumor immune response and must be preceded for antigen presentation, gamma ray was irradiated at 10gy with BMDM differentiated for 2 days and CFSE fluorescence-labeled B16F10 was co-cultured for 24 hours. Subsequently, as a result of measuring CFSE fluorescence expressed in BMDM via flow cytometry, it was found that the phagocytosis in both CD244-deficient monocytes and macrophages increased significantly compared to the control group (WT) (FIG. 4b-I and FIG. 4b-J).

### [Example 5] Analysis of the cause of increased CD244 expression in monocytes upon tumor induction

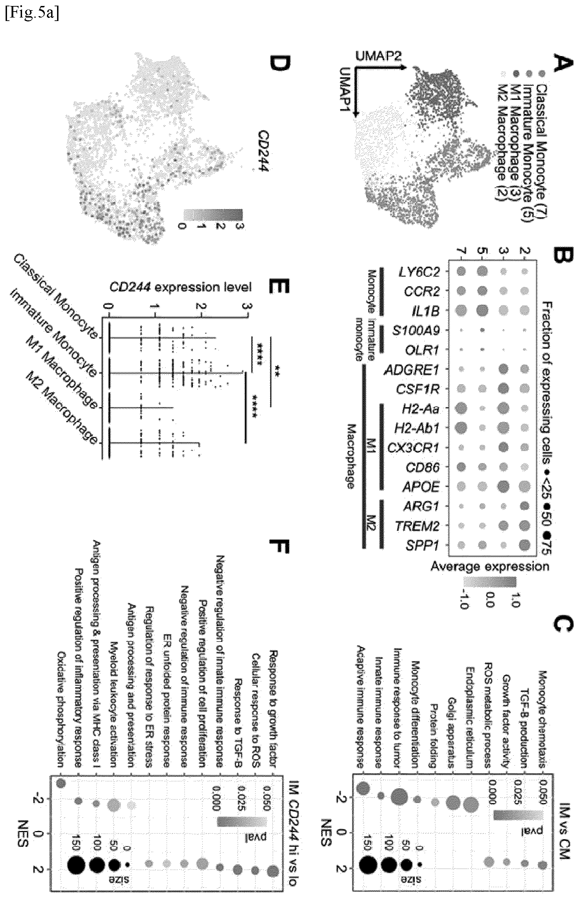

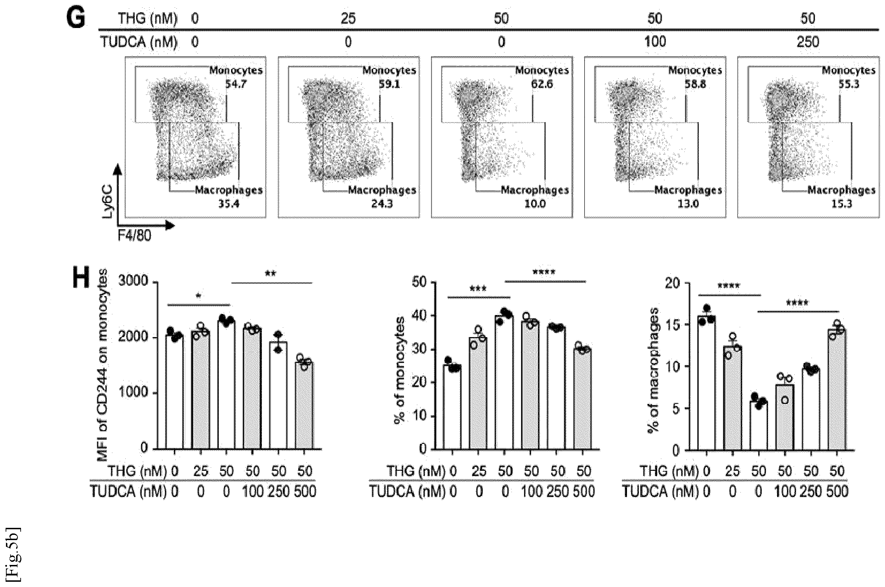

In FIG. 1a-A, in order to determine the cause of elevation in the CD244 expression in monocytes upon tumor induction and the molecular mechanism by which CD244 inhibits differentiation and functions of monocyte-macrophage, analysis was conducted on mouse tumor model single-cell transcriptome analysis data (GSE121861) in public databases. Seven CD45 (PTPRC)+ immune cell clusters were distinguished using representative marker expression patterns, and the expression levels of known markers such as monocytes (LY6C2, CCR2, and IL1B), immature monocytes (S100A9 and OLR1), macrophages (ADGRE1 and CSF1R), M1 (H2-Aa, H2Ab1, CX3CR1, CD86, and APOE), and M2 (ARG1, TREM 2, and SPP1) macrophages were identified to determine subclusters of the four monocyte lineage cells (FIG. 5a-A and FIG. 5a-B). Through the pathway enrichment analysis, it was revealed that immature monocytes expressed genes related to immune response, differentiation, and protein folding at a lower rate than normal monocytes (FIG. 5a-C). As a result of identifying CD244 expression in a subgroup of four monocyte lineage cells, it was determined that the CD244 expression was significantly high in immature monocytes compared to normal monocytes and M1/M2 macrophages (FIG. 5a-D and FIG. 5a-E). In addition, compared to the immature monocytes with low CD244 expression, it was found that the ER stress pathway was upregulated in the immature monocytes with high expression of CD244 (FIG. 5f). Assuming that the ER stress pathway, one of the major mechanisms in the development of immunosuppressive immature monocytes, also regulates CD244 expression in immature monocytes, when BMDM was treated with Thapsigargin which is an ER stress inducer, CD244 expression in monocytes increased and differentiation into macrophages decreased. On the other hand, when treating tauroursodeoxycholic acid, an ER stress inhibitor, it was found that CD244 expression decreased in monocytes while differentiation into macrophages increased (FIG. 5b-G and FIG. 5b-H).

### [Example 6] Analysis of molecular mechanisms modulated by CD244

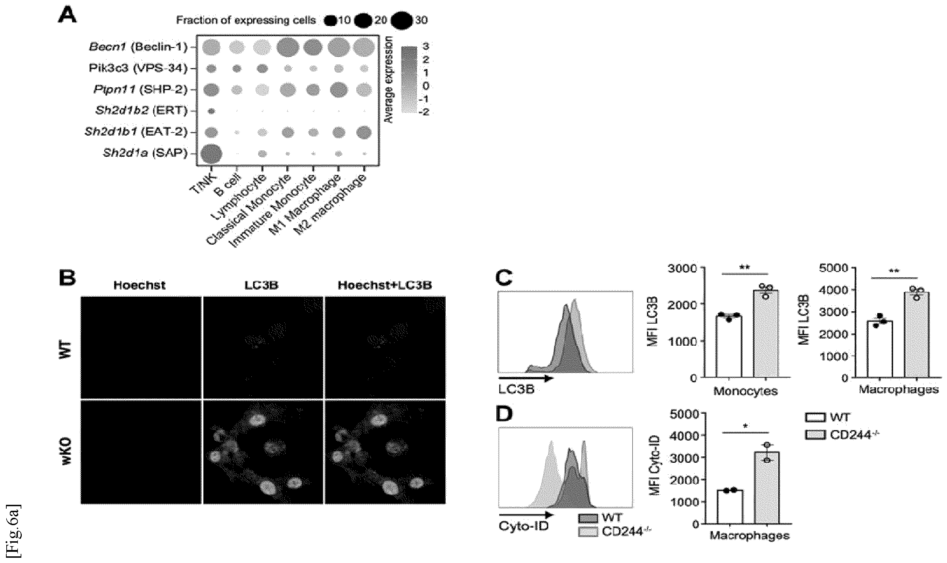

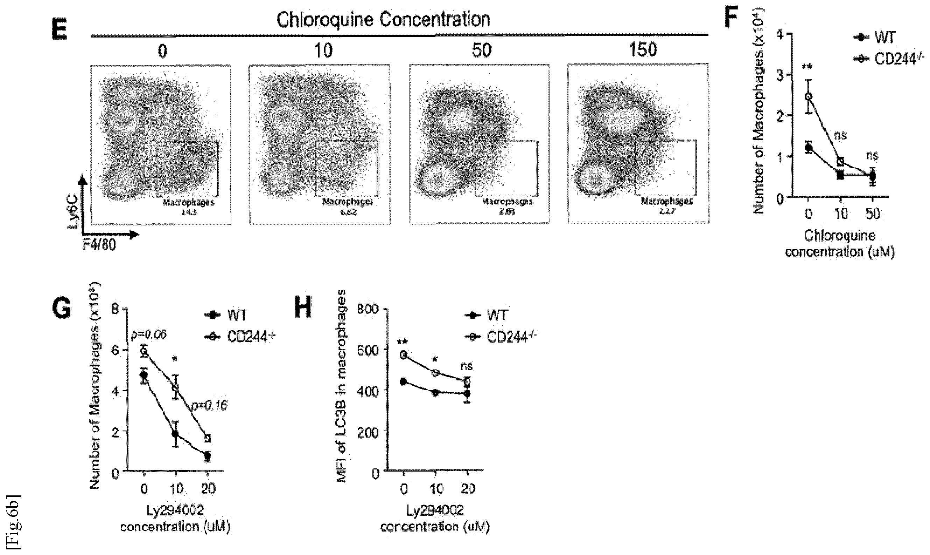

To determine how CD244 inhibits monocyte-macrophage differentiation, antigen presentation, and phagocytosis, the expression of signaling molecules of CD244 was identified in the single-cell transcriptome analysis data of Example 5. As a result, it was found that the expression of SAP, EAT-2, ERT, and SHP-2 was low, and that of Beclin-1 was high in monocyte-macrophages compared to T cells and NK cells (FIG. 6a-A). In addition, the incidence of autophagy in WT and CD244-deficient BMDM was checked. First, in order to identify the lipidation of LC3, which is a process required for autophagy to begin, as a result of staining WT and CD244 KO BMDM with anti-LC3B antibody (abcam: #ab51520) and then observing through fluorescence microscopy and flow cytometry, it was found that LC3 lipidation significantly increased in CD244 KO BMDM (FIG. 6a-B and FIG. 6a-C). It was discovered in the result of quantifying the amount of autophagosomes actually formed by LC3B through cyto-ID staining that there was a significant increase in CD244 KO BMDM (FIG. 6a-D) as well.

Next, to determine whether a degree of differentiation of BMDM changes when autophagy was controlled, autophagy-modulating drugs were treated. In the treatment of chloroquine, which interferes with the fusion of autophagosomes and lysosomes, thereby inhibiting the final stage of autophagy, the number of macrophages in both WT and CD244 KO BMDM was remarkably reduced (FIG. 6b-E and FIG. 6b-F). In addition, even in the treatment of Ly294002, an inhibitor of vps-34, another molecule that binds to CD244, it was found that the number of macrophages decreased, and the difference in LC3B expression between WT and CD244^{-/-} BMDM disappeared (FIG. 6b-G and FIG. 6b-H). From the above results, it was determined that CD244 inhibits the differentiation from monocytes into macrophages by inhibiting autophagy.

### [Example 7] Analysis of an anticancer effect by co-administration of CD244-deficient monocytes/macrophages and anti-PD-L1 antibodies

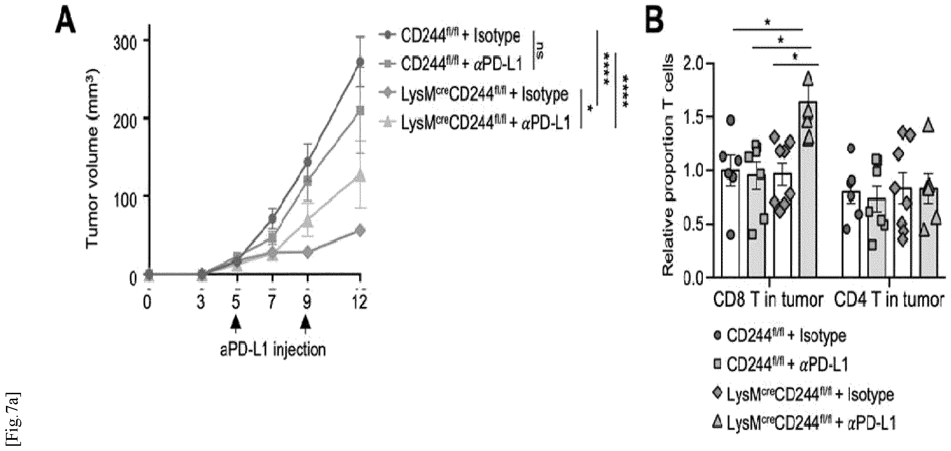

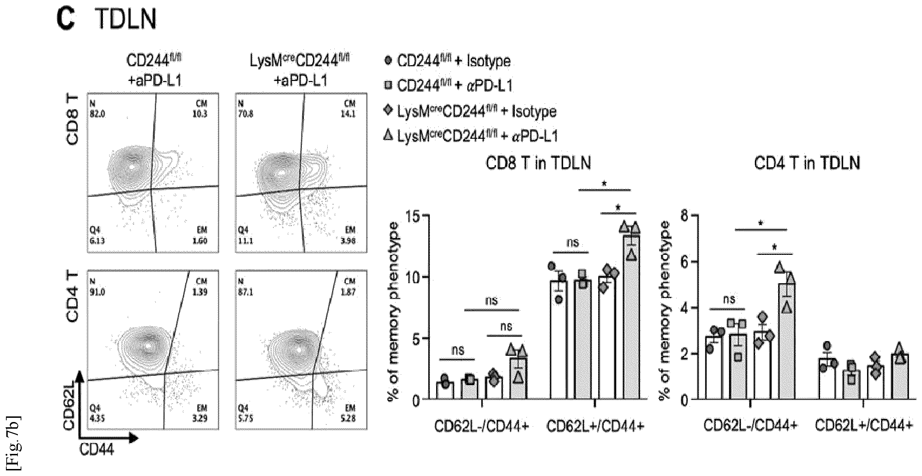

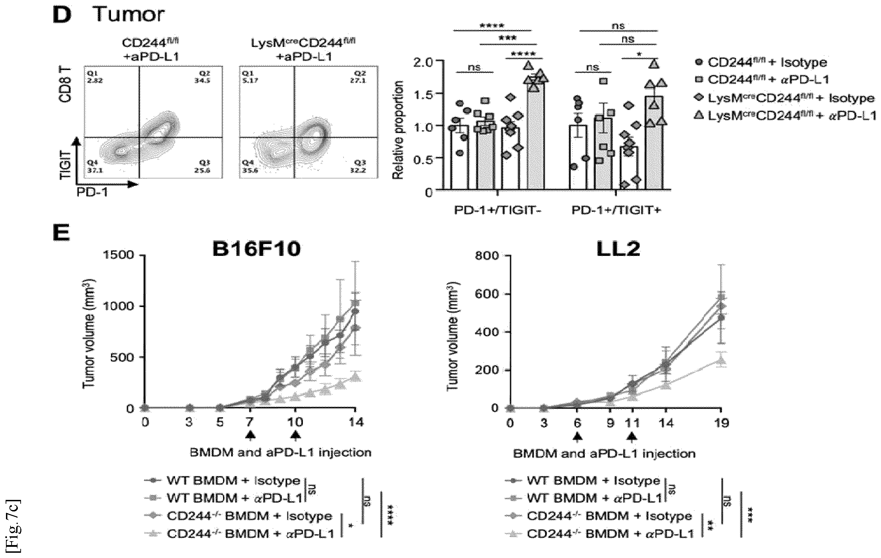

Based on the above results, it was determined that CD244 suppresses the anti-tumor immune response by accumulating monocytes in the tumor microenvironment and subsequently reducing differentiation into macrophages, antigen presentation, and phagocytosis, and thus the experiments were conducted on the possibility of applying CD244-deficient macrophages as a treatment method to immunologically convert "cold" tumors into "hot" tumors. First, B16F10 melanoma was induced in monocyte lineage-specific CD244-deficient mice (cKOs) and control group Flox mice, and then treated with anti-PD-L1 antibodies. As a result, the tumors of Flox mice did not show significant changes in the anti-PD-L1 antibody treatment. However, anti-PD-L1 antibody treatment further reduced tumor size in cKO mice compared to isotype antibody treatment (FIG. 7a-A). When the proportion of intratumor T cells was measured, it was found that the proportion of CD8 T cells increased significantly only when anti-PD-L1 antibodies were treated to cKO mice (FIG. 7a-B). In addition, as a result of analyzing the memory phenotype of T cells in TDLN of cKO mice treated with anti-PD-L1 antibodies, the central memory (CD62L+, CD44+) CD8 T cells and effector memory (CD62L-, CD44+) CD4 T cell populations significant increased compared to when anti-PD-L1 antibodies were treated to the Flox mice (FIG. 7b). In addition, the proportion of T cells expressing PD-1 and not expressing T cell immunoreceptors with Ig and ITIM domains (TIGIT) increased significantly only in tumors of cKO mice injected with anti-PD-L1 antibodies, whereas the proportion of PD-1/TIGIT double positive CD8 T cells showed no significant change (FIG. 7c-D). In general, since it is known that PD-1 is an immune checkpoint receptor that is expressed in both antigen-specifically activated T cells, whereas TIGIT is expressed only in severely exhausted T cells, the above results showed that treatment of anti-PD-L1 antibodies to cKO mice increases the activity of CD8 T cells in tumors but does not increase the immune exhaustion.

To investigate the potential of CD244-deficient macrophages as an anti-tumor immune cell therapeutic drug, WT mice with B16F10 melanoma were co-injected with anti-PD-L1 antibodies and CD244^{-/-} BMDM cells. When isotype antibody and CD244^{-/-} BMDM were treated together, tumor growth was slightly inhibited, but co-administration of CD244^{-/-} BMDM and anti-PD-L1 antibody significantly reduced the growth of B16F10 tumors, and the same results were observed in another cold tumor model, Lewis lung carcinoma (LL2) (FIG. 7c-E). From the above result, CD244-deficient macrophages increase T cell activity and memory phenotype, increase intratumor CD8 T cells, and enhance responsiveness to immune checkpoint inhibitors in tumor models, including melanoma.

### [Example 8] Analysis of an anticancer effect of CD244-deficient monocytes/macrophages on melanoma patients

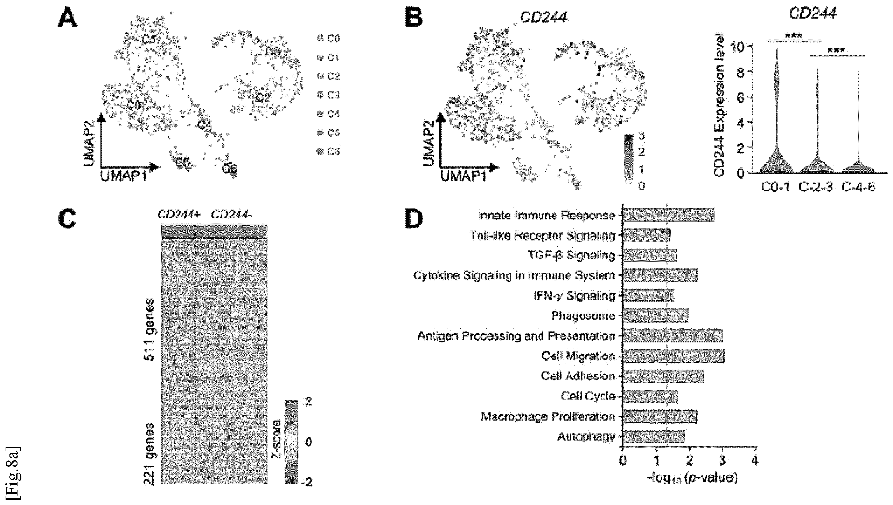

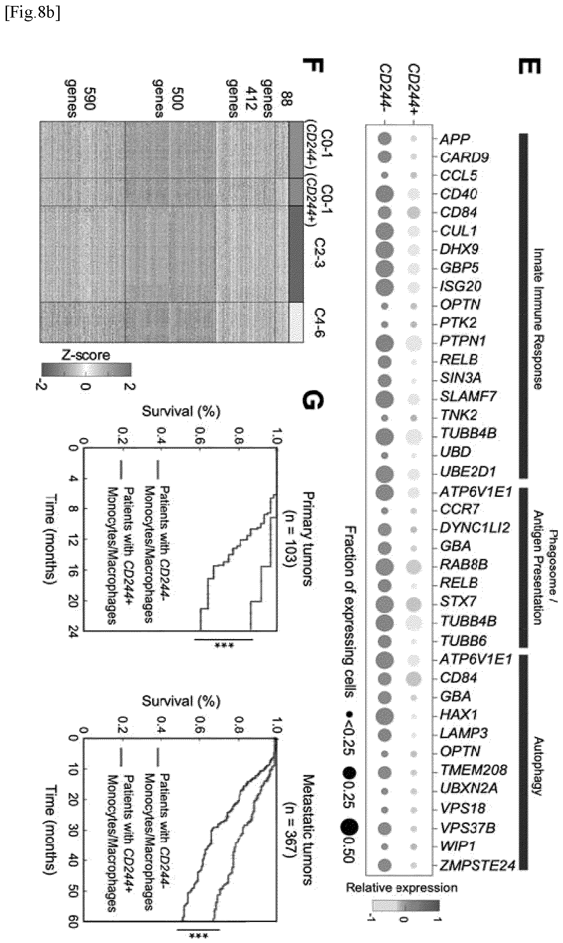

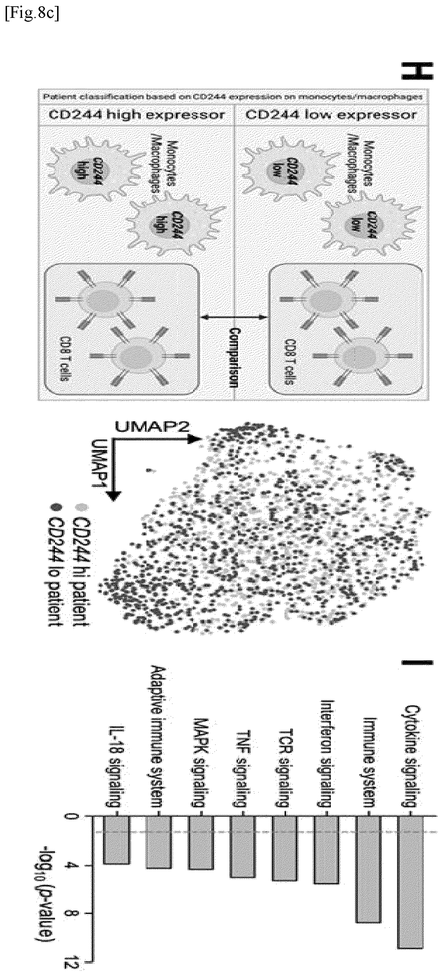

To determine the clinical significance of CD244 as an immune checkpoint receptor for monocytes/macrophages in the tumor microenvironment, a single cell RNA sequencing dataset (SCP398) of CD45+ immune cells isolated from human melanoma tissues from a public database was analyzed. Of the 16,291 cells reported in the previous study, 1,391 cells classified as monocytes and macrophages were used. To identify monocytes/macrophages that do not express CD244 among these cells, they were further classified into seven subgroups using the shared nearest neighbor clustering method in Seurat (FIG. 8a-A). They were then classified into three groups according to CD244 expression: 1) C0-1 with a relatively high proportion of CD244-expressing cells, 2) C2-3 with a relatively low proportion of CD244-expressing cells, and 3) C4-6 with no CD244 expression (FIG. 8a-B). Of these groups, C0-1 with a large number of CD244+ and CD244-monocytes/macrophages was used for a fair comparison between the two cell types. A total of 511 genes and 221 genes were found to be up-and down-regulated in CD244+ and CD244-monocytes/macrophages, respectively (FIG. 8a-C). The 221 genes expressed primarily in CD244-monocytes/macrophages were highly associated with phagosomes, antigen processing and presentation, and autophagy (FIG. 8a-D), consistent with the findings in the mouse models. Next, an attempt was made to determine whether the degree of expression of CD244 in monocytes/macrophages had an impact on patient survival rate. To this end, a bulk RNA-seq dataset generated from tumor tissues from 470 melanoma patients (103 primary tumors and 367 metastatic tumors) was obtained from The Cancer Genome Atlas database (TCGA-SKCM, FIG. 8b-E). Then, in the bulk RNA-seq dataset, in order to estimate the proportion of the four cell types identified in the preceding scRNA-seq analysis, specifically upregulated genes (DEGs) were identified in the four cell types, and the TCGA dataset was subjected to the deconvolution analysis via CIBERSORTx (FIG. 8b-F). By comparing the survival rate of patients with and without monocytes/macrophages with no CD244 expression, it was found that patients with monocytes/macrophages with no CD244 expression had remarkably higher overall survival rate than those without in both primary and metastatic cancer cohorts (FIG. 8b-G).

Next, it was intended to determine whether monocytes/macrophages with low CD244 expression are able to enhance antigen-specific activity of T cells even in humans, as in the mouse models. In the preceding scRNA-seq data set, individual patients were classified into CD244 high and CD244 low groups according to the mean expression level of CD244 in monocytes/macrophages, and CD8 T cells were compared between CD244 high and low groups regardless of CD244 expression in T cells (FIG. 8c-H). Thereby, 207 genes were obtained that were mainly upregulated in CD8 T cells in the CD244 low group, revealing that these genes were mainly associated with cytokine signaling, TCR signaling, and the adaptive immune system (FIG. 8c-I).

### [Example 9] Analysis of an effect of CD244 on the function of monocytes-macrophages in different carcinomas

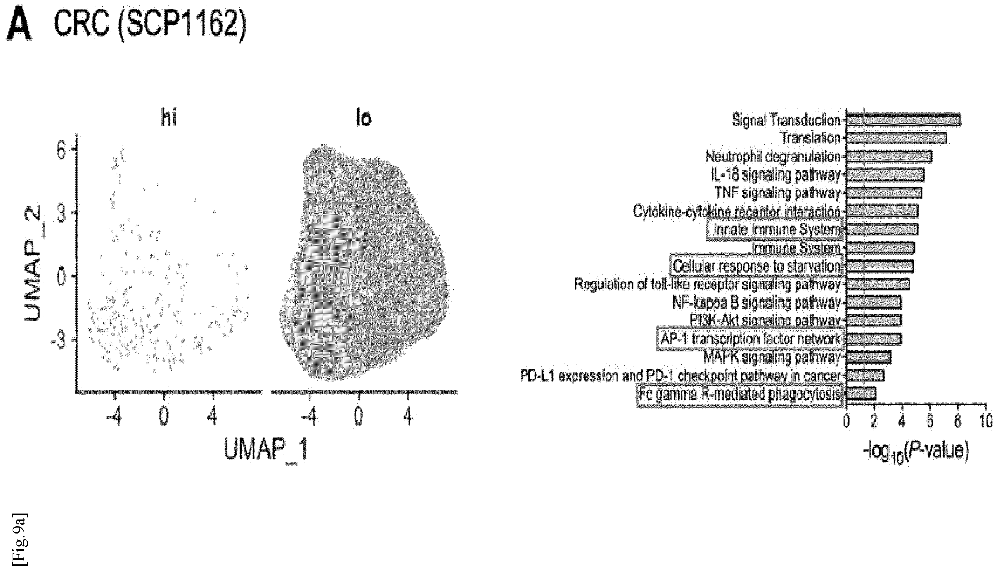

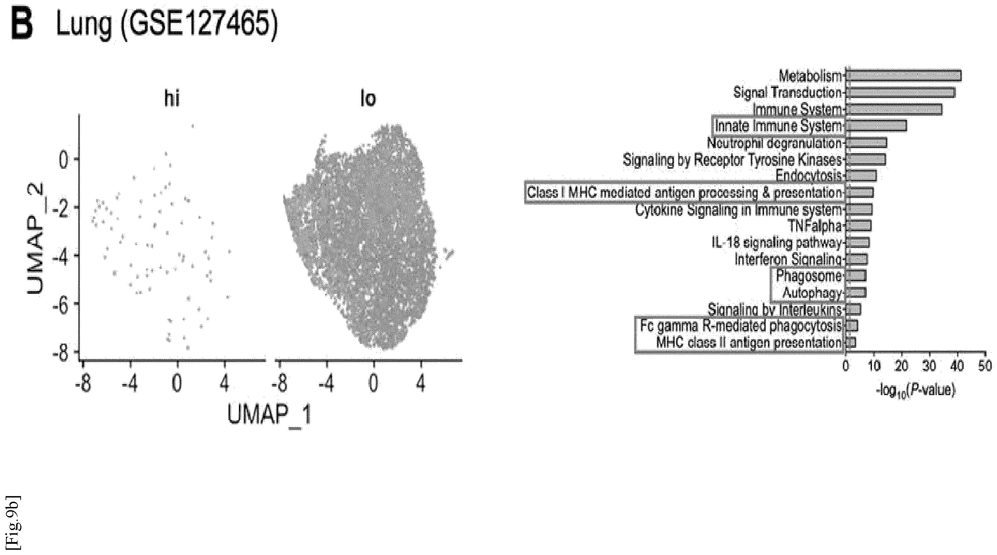

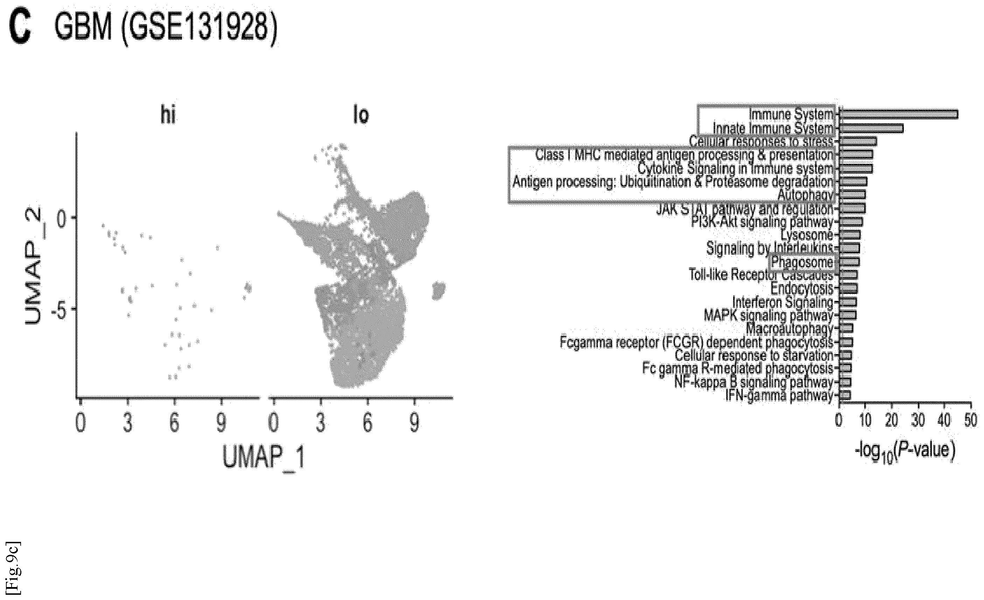

Finally, checked was whether the results for CD244 expressed in monocyte-based cells identified from melanoma models and transcriptome analysis data could be extended to other tumor models. To this end, single-cell transcriptome analysis datasets of patients with colorectal cancer (SCP1162) (FIG. 9a), non-small cell lung cancer (GSE127465) (FIG. 9b), and glioblastoma (GSE131928) (FIG. 9c) were analyzed. As a result of conducting a pathway enrichment analysis using DEG of monocytes/macrophages that do not express CD244, it was found that CD244 regulates the innate immune system, phagocytosis, antigen presentation, and autophagy in monocyte-macrophages in carcinomas other than melanoma.

While a specific part of the present disclosure has been described in detail above, it is clear for those skilled in the art that this specific description is merely preferred example embodiments, and the scope of the present disclosure is not limited thereby. In other words, the substantial scope of the present disclosure is defined by the appended claims and their equivalents.

## Claims

1. A pharmaceutical composition for preventing or treating a cancer, comprising, as an active ingredient, monocytes or macrophages with suppressed CD244 expression or activity.

2. The pharmaceutical composition of claim 1, wherein the pharmaceutical composition inhibits cancer growth by increasing phagocytosis or antigen presentation.

3. The pharmaceutical composition of claim 1, wherein the pharmaceutical composition increases expression of IFN-g by inducing antigen-specific activation of T cells.

4. The pharmaceutical composition of claim 1, wherein the monocyte comprises one or more markers selected from the group consisting of CD11b+, CD14+, CD16-, Ly6G-, and Ly6Chi.

5. The pharmaceutical composition of claim 1, wherein the macrophage comprises one or more markers selected from the group consisting of CD11b+, CD14+, CD16+, CD68+, Ly6G-, Ly6Clo, and F4/80+.

6. The pharmaceutical composition of claim 1, wherein the cancer is one or more selected from the group consisting of stomach cancer, colon cancer, breast cancer, lung cancer, non-small cell lung cancer, bone cancer, pancreatic cancer, skin cancer, head cancer, head and neck cancer, melanoma, uterine cancer, ovarian cancer, colon cancer, small intestine cancer, rectal cancer, anal cancer, fallopian tube cancer, endometrial cancer, cervical cancer, vaginal cancer, vulvar cancer, Hodgkin's disease, esophageal cancer, lymphoma, bladder cancer, gallbladder cancer, endocrine cancer, prostate cancer, adrenal cancer, soft tissue sarcoma, urethral cancer, penile cancer, chronic or acute leukemia, lymphocytic lymphoma, renal cancer, ureteral cancer, renal pelvic cancer, blood cancer, brain cancer, central nervous system (CNS) tumors, spinal cord tumors, brainstem gliomas, and pituitary adenomas.

7. A pharmaceutical composition for co-administration for preventing or treating a cancer, comprising: monocytes or macrophages with suppressed CD244 expression or activity; and an anticancer drug as active ingredients.

8. The pharmaceutical composition of claim 7, wherein the anticancer drug is one or more anticancer drugs selected from the group consisting of a cytotoxic anticancer drug, a targeted anticancer drug, and an immunotherapeutic drug.

9. The pharmaceutical composition of claim 8, wherein the cytotoxic anticancer drug is one or more selected from the group consisting of doxorubicin, cyclophosphamide, temozolomide, irinotecan, cisplatin, vinblastine, vincristine, actinomycin-D, 5-fluorouracil, docetaxel, cabazitaxel, paclitaxel, and pembrolizumab.

10. The pharmaceutical composition of claim 8, wherein the targeted anticancer drug is one or more selected from the group consisting of trametinib, vemurafenib, alpelisib, dactolisib, Gefitinib, Erlotinib, Lapatinib, Sunitinib, Sorafenib, Crizotinib, Dabrafenib, Trastuzumab, Cetuximab, Bevacizumab, Panitumumab, Ipilimumab, Pertuzumab, Tofacitinib, Imatinib, Bortezomib, Ofatumumab, and Alemtuzumab.

11. The pharmaceutical composition of claim 8, wherein the immunotherapeutic drug is one or more selected from the group consisting of anti-PD-1, anti-PD-L1, anti-CTLA-4, anti-LAG-3, anti-TIGIT, anti-TIM-3, anti-GITR, CAR-T, and NK cells.

12. A composition for enhancing efficacy of an anticancer drug, comprising, as an active ingredient, monocytes or macrophages with suppressed CD244 expression or activity.

13. A method of preventing or treating a cancer, comprising administrating monocytes or macrophages with suppressed CD244 expression or activity to a subject, including a human, alone or in combination with an anticancer drug.

14. A composition for inducing differentiation from monocytes to macrophages, comprising a CD244 inhibitor or an inhibitor of CD48 which is a ligand of CD244 as an active ingredient.

15. The composition of claim 14, wherein the CD244 inhibitor or the CD48 inhibitor is selected from the group consisting of compounds, peptides, peptide mimetics, aptamers, antibodies, and natural substances that specifically bind to a CD244 or CD48 protein, or selected from the group consisting of small interfering RNAs (siRNAs), antisense nucleotides, and short hairpin RNAs (shRNAs) that complementarily bind to an mRNA of a CD244 or CD48 gene.

16. A method of screening an inducer for differentiation from monocytes to macrophages, comprising treating cells with candidate substances to analyze suppression of expression or activity of CD244 or CD48 which is a ligand thereof.
